# DEMANDE DE BREVET EUROPEEN

(11) **EP 3 858 467 A1**
(43) Date de publication de la demande: **04.08.2021**
(21) Numéro de dépôt: 21151531.7
(22) Date de dépôt: 14.01.2021
(51) Int. Cl.: B01D 53/22, C12M 1/00

(54) **INSTALLATION ET PROCEDE POUR LA PRODUCTION DE BIOGAZ DESULFURE**

(30) Priorité: 31.01.2020 FR 2000971
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: BERTRANDIAS, Aude, 78350 Les Loges-En-Josas (FR); VALENTIN, Solène, 38360 Sassenage (FR)
(74) Mandataire: Air Liquide

(57) **Abrégé**

Dispositif de production de biogaz au moins partiellement désulfuré comprenant :
- un digesteur,
- un moyen d'introduction en continu de biochar dans le digesteur et
- une unité de séparation membranaire placée sur le flux de biogaz sortant du digesteur et comprenant au moins une membrane laissant perméer les produits soufrés.

## Description

La présente invention est relative à une installation et un procédé pour la production de biogaz désulfuré ou à teneur réduite en H2S.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, et dont les conditions sont contrôlées, appelé méthaniseur ou digesteur, puis dans un post-digesteur, similaire au digesteur et permettant de pousser plus loin la réaction de méthanisation.

On appellera biomasse tout groupement de matières organiques pouvant se transformer en énergie à travers ce processus de méthanisation e.g. boues de station d'épuration, fumiers/lisiers, résidus agricoles, déchets alimentaires...

Le digesteur, c'est-à-dire le réacteur dédié à la méthanisation de la biomasse, est une cuve fermée, chauffée ou non (opération à une température fixée, entre la température ambiante et 55°C) et dont le contenu constitué de la biomasse est brassé, en continu ou séquentiel. Les conditions dans le digesteur sont anaérobies et le biogaz généré se retrouve dans l'espace de tête du digesteur (ciel gazeux), où il est prélevé. Les post-digesteurs sont similaires aux digesteurs.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles. Le biogaz contient majoritairement du méthane (CH4) et du dioxyde de carbone (CO2) dans des proportions variables en fonction du mode d'obtention et du substrat mais peut également contenir, en moindres proportions de l'eau, de l'azote, de l'hydrogène sulfuré (H2S), de l'oxygène, ainsi que des composés organiques autres, à l'état de traces, , dont le H2S, entre 10 et 50,000 ppmv..

Selon les matières organiques dégradées et les techniques utilisées, les proportions des composants diffèrent, mais en moyenne le biogaz comporte, sur gaz sec, de 30 à 75% de méthane, de 15 à 60% de CO2, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au cœur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (bioGNL)...

Selon la composition de la biomasse, le biogaz produit lors de la digestion contient du sulfure d'hydrogène (H2S) dans des teneurs comprises entre 50 et 50 000 ppm. Quelle que soit la destination finale de valorisation du biogaz, il s'avère indispensable d'éliminer le sulfure d'hydrogène qui est une impureté toxique et corrosive. De plus, si l'utilisation du biogaz consiste à l'épurer pour injecter du biométhane dans le réseau de gaz naturel, des spécifications strictes limitent la quantité de H2S autorisée. Plusieurs méthodes d'élimination du H2S existent et sont plus ou moins répandues (lits de charbon actif, ajout de composés de fer, absorption physique ou chimique, lavages à l'eau, biofiltres...). L'élimination se fait principalement par adsorption sur lit de charbon actif, à l'extérieur du digesteur. Dans un nombre croissant de digesteurs, l'abattement de l'H2S se fait aussi en partie par injection d'air/air enrichi/O2 dans le ciel gazeux du digesteur, ce qui constitue une solution in situ. Avec une injection dans le ciel gazeux à faible dose, du soufre solide est formé à partir du H2S et O2 (eq. (1)), réalisée par des bactéries sulfo-oxydantes e.g. Thiobacillus. A haute dose d'O2 injecté, le milieu est acidifié (eq. (2)). La réaction (1) est donc ciblée.

H2S + 0.5 02 → S + H2O (1)

H2S + 2 O2 → SO4 2- + 2 H + (2)

Les quantités d'injection d'O2 nécessaires en pratiques sont différentes de celles attendues par la stoechiométrie de l'éq. (1): des doses de 0.3 - 3% d'O2 par rapport au biogaz généré sont le plus souvent recommandées, avec des doses jusqu'à 12% qui sont parfois évoquées. Aujourd'hui, l'injection d'air/air enrichi/O2 in situ n'est pas optimisée et les lits de charbon actif doivent donc être maintenus pour éliminer la totalité du H2S.

Partant de là, un problème qui se pose est de fournir une installation améliorée favorisant l'élimination plus poussée de l'H2S.

Une solution de la présente invention est une installation de production de biogaz au moins partiellement désulfuré comprenant :
- un digesteur,
- un moyen d'introduction en continu de biochar dans le digesteur et
- une unité de séparation membranaire placée sur le flux de biogaz sortant du digesteur et comprenant au moins une membrane laissant perméer les produits soufrés.

Le biochar est du charbon actif produit par pyrolyse en condition limitée en oxygène d'une biomasse végétale de type bois, riz, écorce de noix de coco, etc, ...

Le biochar est introduit dans la phase liquide du digesteur, afin d'adsorber l'hydrogène sulfurée. L'adsorption est effective en présence d'oxygène. Si le biochar et l'oxygène sont introduits dans les bonnes proportions, l'élimination de l'hydrogène sulfurée peut être complète.

Le biochar ayant adsorbé l'hydrogène sulfuré est évacué en même temps que les digestats et permet d'augmenter la capacité nutritive des sols du digestat.

La présente invention propose de coupler l'utilisation du biochar à l'intérieur du digesteur avec une membrane laissant perméée les produits soufrés, afin d'éliminer l'utilisation de lits de charbons actifs pour l'épuration du biogaz en dehors du digesteur. Le perméat gazeux sera recyclé dans le digesteur, afin d'être traité à l'intérieur du digesteur par le biochar introduit.

Selon le cas l'installation selon l'invention peut présenter une ou plusieurs des caractéristiques ci-dessous :
- le digesteur comprend un système de brassage mécanique, pneumatique ou hydraulique.
- le dispositif comprend un système d'extraction et de pressage du digestat produit simultanément avec le biogaz.
- Le dispositif comprend un moyen d'introduction d'un gaz d'oxydation permettant d'atteindre une teneur en oxygène dans le ciel gazeux comprise entre 0,3 et 3%.
- Le dispositif comprend à la sortie du digesteur successivement dans le sens de circulation du flux de biogaz un surpresseur, un sécheur, un compresseur et l'unité de séparation membranaire.
- le surpresseur permet d'augmenter la pression du biogaz à une pression comprise entre 1 et 2 bar, de préférence entre 1 et 1,5 bar, encore plus préférentiellement entre 1 et 1,3 bar.
- le sécheur est à une température comprise entre 3 et 7°C, de préférence à 5°C.
- le compresseur permet d'augmenter la pression du biogaz à une pression comprise entre 5 et 20 bar, de préférence entre 8 et 15 bar.

La présente invention a également pour objet un procédé de production de biogaz au moins partiellement désulfuré mettant en œuvre un dispositif selon l'invention comprenant les étapes suivantes :
a) Introduction en continu du biochar dans le digesteur ;
b) Introduction de la biomasse dans le digesteur ;
c) Digestion anaérobie de la biomasse dans le digesteur et production de biogaz;
d) Séparation du biogaz et du digestat ;
e) Séparation des produits soufrés contenus dans le biogaz par voie membranaire dans l'unité de séparation membranaire ; et
f) Récupération d'un flux de biogaz au moins partiellement désulfuré.

Selon le cas, le procédé selon l'invention peut présenter une ou plusieurs des caractéristiques ci-dessous :
- les étapes a) et b) sont réalisées simultanément ;
- l'étape a) est réalisée avant l'étape b) ;
- le biochar présente une granulométrie comprise entre 1 µm et 6 µm ;
- la quantité de biochar introduite dans le digesteur correspond à chaque instant t du procédé à une valeur comprise entre 5 et 15 % en poids de la quantité de biomasse introduite dans le digesteur.
- le procédé comprend entre les étapes b) et c) une étape de brassage du biochar et de la biomasse dans le digesteur.
- la biomasse comprend moins de 15% de matière sèche.
- le biochar a un pH alcalin supérieur à 7, de préférence supérieur à 9, encore plus préférentiellement supérieur à 11.

La solution selon l'invention permet d'obtenir un flux de biogaz en sortie de l'unité de séparation membranaire comprenant moins de 50 ppm de sulfure d'hydrogène. Le digestat pourra servir pour l'épandage au sol.

L'invention permet de réduire le coût d'épuration du sulfure d'hydrogène du biogaz de façon efficace, en augmentant la réactivité de l'oxygène déjà injecté avec les produits soufrés grâce à l'introduction de biochar dans la partie liquide du digesteur et en éliminant les lits de charbons actifs traitant le biogaz gazeux grâce à la perméation des produits soufrés restant et à leur recycle en tête du digesteur.

## Revendications

1. Dispositif de production de biogaz au moins partiellement désulfuré comprenant :
- un digesteur,
- un moyen d'introduction en continu de biochar dans le digesteur et
- une unité de séparation membranaire placée sur le flux de biogaz sortant du digesteur et comprenant au moins une membrane laissant perméer les produits soufrés.

2. Dispositif selon la revendication 1, **caractérisé en ce que** le digesteur comprend un système de brassage mécanique, pneumatique ou hydraulique.

3. Dispositif selon l'une des revendications 1 ou 2, **caractérisé en ce qu'**il comprend un système d'extraction et de pressage du digestat produit simultanément avec le biogaz.

4. Dispositif selon l'une des revendications 1 à 3, **caractérisé en ce qu'**il comprend un moyen d'introduction d'un gaz d'oxydation permettant d'atteindre une teneur en oxygène dans le ciel gazeux comprise entre 0,3 et 3%.

5. Dispositif selon l'une des revendications 1 à 4, **caractérisé en ce qu'**il comprend à la sortie du digesteur successivement dans le sens de circulation du flux de biogaz un surpresseur, un sécheur, un compresseur et l'unité de séparation membranaire.

6. Dispositif selon la revendication 5, **caractérisé en ce que** le surpresseur permet d'augmenter la pression du biogaz à une pression comprise entre 1 et 2 bar, de préférence entre 1 et 1,5 bar, encore plus préférentiellement entre 1 et 1,3 bar.

7. Dispositif selon l'une des revendications 5 et 6, **caractérisé en ce que** le sécheur est à une température comprise entre 3 et 7°C, de préférence à 5°C.

8. Dispositif selon l'une des revendications 5 à 7, **caractérisé en ce que** le compresseur permet d'augmenter la pression du biogaz à une pression comprise entre 5 et 20 bar, de préférence entre 8 et 15 bar.

9. Procédé de production de biogaz au moins partiellement désulfuré mettant en œuvre un dispositif selon l'une des revendications 1 à 8 comprenant les étapes suivantes :
a) Introduction en continu du biochar dans le digesteur ;
b) Introduction de la biomasse dans le digesteur ;
c) Digestion anaérobie de la biomasse dans le digesteur et production de biogaz;
d) Séparation du biogaz et du digestat ;
e) Séparation des produits soufrés contenus dans le biogaz par voie membranaire ; et
f) Récupération d'un flux de biogaz au moins partiellement désulfuré.

10. Procédé de production de biogaz selon la revendication 9, **caractérisé en ce que** les étapes a) et b) sont réalisées simultanément.

11. Procédé de production de biogaz selon la revendication 9, **caractérisé en ce que** l'étape a) est réalisée avant l'étape b).

12. Procédé de production de biogaz selon l'une des revendications 5 à 6, **caractérisé en ce que** le biochar présente une granulométrie comprise entre 1 µm et 6 µm.

13. Procédé selon l'une des revendications 9 à 12, **caractérisé en ce que** la quantité de biochar introduite dans le digesteur correspond à chaque instant t du procédé à une valeur comprise entre 5 et 15 % en poids de la quantité de biomasse introduite dans le digesteur.

14. Procédé selon l'une des revendications 9 à 13, **caractérisé en ce qu'**il comprend entre les étapes b) et c) une étape de brassage du biochar et de la biomasse dans le digesteur.

15. Procédé selon l'une des revendications 9 à 14, **caractérisé en ce que** la biomasse comprend moins de 15% de matière sèche.

16. Procédé selon l'une des revendications 9 à 15, **caractérisé en ce que** le biochar a un pH alcalin supérieur à 7, de préférence supérieur à 9, encore plus préférentiellement supérieur à 11.
